# EUROPEAN PATENT SPECIFICATION

(11) **EP 1 210 312 B1**
(45) Date of publication and mention of the grant of the patent: **28.04.2004**
(21) Application number: 00957330.4
(22) Date of filing: 08.08.2000
(51) Int. Cl.: C07C 51/265, C07C 51/41, C07C 51/487

(54) **CLOSELY LINKING A NDA PROCESS WITH A PEN PROCESS**
ENG GEKOPPELTES 2,6-NAPHTHALENEDICARBONSÄURE-VERFAHREN MIT EINEM POLYETHYLENNAPHTHALAT-VERFAHREN
ASSOCIATION ETROITE D'UN PROCEDE NDA A UN PROCEDE PEN

(30) Priority: 30.08.1999 US 151603 P
(43) Date of publication of application: 05.06.2002
(73) Proprietor: Mossi & Ghisolfi International S.A., 2320 Luxembourg (LU)
(72) Inventor: RODDEN, John, B., Houston, TX 77059 (US); ELLIOTT, Glenn, W., Akron, OH 44313 (US)
(74) Representative: Gerbino, Angelo
(86) International application number: PCT/US2000/021670
(87) International publication number: WO 2001/016066

(56) References cited:
- EP-A- 0 926 124
- US-A- 3 671 578
- US-A- 4 820 868
- US-A- 5 292 934
- DATABASE WPI Derwent Publications Ltd., London, GB; AN 1974-03789v XP002160524 "2,6-naphthalene dicarboxylic acid salt - preparation from crude naphthoic acid" & JP 49 000828 B (TEIJIN LTD) 10 January 1974 (1974-01-10)
- DATABASE WPI Derwent Publications Ltd., London, GB; AN 1995-203774 XP002160525 "Purification of 2,6-naphthalene dicarboxylic acid" & JP 07 118201 A (MITSUBISHI PETROCHEMICAL CO LTD) 9 May 1995 (1995-05-09)

## Description

### Technical Field

This invention relates to the production of 2,6-naphthalene dicarboxylic acid (hereinafter abbreviated as 2,6-NDA) and to the production of polyethylene naphthalate (hereinafter abbreviated as PEN). More particularly this invention makes it possible for the first time to eliminate the drying and solids handling portions of a process for preparing 2,6-NDA and pump the 2,6-NDA in an aqueous slurry directly into to a process for making PEN. This invention also relates to the preparation of 2,6-NDA pure enough for polymerization.

### Background Art

Films, fibers and other shaped articles prepared from PEN display improved strength and thermal properties relative to other polyester materials. High strength fibers made from PEN can be used to make tire cords and films made from PEN are advantageously used to manufacture magnetic recording tape and components for electronic applications. In comparison with polyethylene terephthalate, PEN is excellent, for example, in mechanical strength and heat stability. PEN is used for films for magnetic tapes, for films for packaging, and for condensers. In recent years it has been used for photograph supports because of its dimensional stability in the form of a thin film.

2,6-NDA and ethylene glycol are the raw materials for producing PEN. The methods for producing PEN are the esterification process and the direct polymerization process, each of which can be carried out batchwise or continuously. Currently in the art the esterification process is more commonly used, but usually employs as the raw material a salt of 2,6-NDA, 2,6-naphthalene dicarboxylate (hereinafter abbreviated 2,6-NDC). 2,6-NDC is often in crystal form with impurities trapped in the structure. 2,6-NDC is used, because there have been no processes available in the art to produce polymerization grade 2,6-NDA, the preferred monomer for making PEN. The availability of a process for making polymer grade 2,6-NDA would make it possible to pursue the preferred route to PEN. This would represent a revolutionary advance in the art. PEN produced by such a process would be much more economical.

It is also known in the art that all previous processes for producing NDA and NDC deliver a solid product that is usually shipped to the polymer manufacturing plant. Though 2,6-NDA is the preferred monomer, handling of NDA particles is still difficult and expensive. In addition, particle size can be critical where dry handling of solids is practiced.

U. S. 4,755,587, for example, discusses the problem of handling solids and claims advantages using very small porous pellets.

Copending EP-A-1 212 277 filed of even date discloses a process for producing 2,6-NDA of polymer grade. The new process is unique in many respects. Of particular importance, the new process can operate using relatively impure methylnaphthalene feedstock with respect to organic hydrocarbon impurities, allows for debromination of the oxidation product in the liquid phase, and avoids the isolation of purified naphthoic acid.

The advent of a process for making polymer grade 2,6-NDA, suitable for direct use in a PEN process without esterification, or solids drying and handling and the associated problems of particle size control is of tremendous value in the art. Such a process constitutes a very significant advance in the art, is much more economical, and is capable of producing polymer grade 2,6-NDA that could be slurried directly into a PEN process. This eliminates problems with solids handling, including the major expense of transporting the 2,6-naphthalene dicarboxylate solids. This constitutes a tremendous advance in the art.

### Disclosure of the Invention

In accordance with the foregoing the present invention is a process for close coupling of a process for making 2,6-NDA and a process for making PEN that has not previously been possible in the art. The invention comprises directly pumping an aqueous slurry of 2,6-NDA, generated in a process for producing 2,6 NDA, into a process for producing PEN. Alternatively, the invention also comprises adding water to 2,6-NDA of polymer quality, which may or may not be already wetted with water, and pumping the resulting slurry of 2,6-NDA directly into a process for producing PEN.

The process comprises:
Pumping an aqueous slurry of polymer grade 2,6-NDA directly into a process for making PEN, either by directly pumping a stream from a 2,6 NDA process into a PEN process, or by adding water to polymer grade 2-6 - NDA prior to pumping the resulting slurry to the PEN process.
Removing the slurry water during the first esterification reaction at the same time the water produced by the esterification reaction is removed.

The following advantages are realized by the disclosed process:
The process allows for the elimination of costly drying equipment that would otherwise be associated with the final steps of the 2-6 NDA process. Through the invention, equipment typically required in the PEN process, such as dense phase or paste make-up facilities, is also eliminated.
Through the inclusion of additional water in the first stage of esterification, the final PEN product is expected to be very low in diethylene glycol concentration. Diethylene glycol is a known contaminant in PEN.

### Detailed Description of the Invention

In the process of the present invention, the 2,6-NDA is a grade that can be used directly in polymerization of PEN. Previously in the art there has not been any process to produce 2,6-NDA monomer of this purity through the use of a final aqueous purification step which uses a reasonable amount of water at moderate temperature and pressure conditions. However, copending U. S. Ser. No. 60/151,577 claims an integrated process for producing 2,6-NDA of the requisite quality and purity which comprises:
a) Reacting a hydrocarbon stream containing predominantly methylnaphthalene with an oxygen-containing gas in the presence of a suitable solvent and catalyst to form a crude mixture of naphthoic acid (crude product NA), wherein said crude product NA remains dissolved in the solvent;
b) Recovering said crude product NA by evaporation of solvent and washing said crude product NA with water;
c) Debrominating said crude product NA by passing over a supported catalyst in the presence of hydrogen, and water-washing said crude debrominated product NA;
d) Contacting said crude, debrominated product NA with an aqueous base of potassium to extract pure NA as the aqueous potassium salt of NA;
e) Separating said aqueous potassium salt of NA from the remaining organic liquid (containing methylnaphthalene and partially oxidized reaction intermediates), and recycling said organic liquid to step a);
f) Contacting said aqueous potassium salt of NA with naphthalene vapor, adding a solid catalyst, and removing water by evaporation to form a slurry of solid potassium salt of NA and catalyst suspended in liquid naphthalene;
g) Reacting said slurry in the presence of carbon dioxide to convert solid potassium salt of NA to liquid naphthalene and solid dipotassium salt of 2,6-NDA(2,6-K2NDA);
h) Reducing the pressure to vaporize the naphthalene, and separating the solids from the naphthalene vapor by a novel separation using cyclones, recycling a portion of the naphthalene to step (f), and recovering the remainder as a product, or methylating the naphthalene via direct alkylation or transalkylation to provide additional methylnaphthalene feed for step (a);
i) Contacting the solids with water to create a mixture of aqueous potassium salts (comprising the potassium salt of NA, KNA, and the dipotassium salt of 2,6-NDA, 2,6 -K2NDA, and its isomers) and solid catalyst;
j) Separating the solid catalyst from the mixture of aqueous potassium salts and recycling it to step (f);
k) Adding aqueous potassium bicarbonate to the mixture of aqueous potassium salts and evaporating a portion of the water to selectively crystallize the dipotassium salt of 2,6-NDA as a solid, separating said solid, and recycling the remaining liquid to step (d);
l) Dissolving the solid dipotassium salt of 2,6-NDA in water;
m) Optionally passing said aqueous dipotassium salt of 2,6-NDA through an activated carbon bed to remove impurities;
n) Contacting said aqueous dipotassium salt of 2,6-NDA with carbon dioxide to create a mixture of solid monopotassium salt of 2,6-NDA and aqueous potassium bicarbonate, separating said solids, and recycling the aqueous potassium bicarbonate to step (k);
o) Contacting solid monopotassium salt of 2,6-NDA with water, optionally in the presence of carbon dioxide, to form solid 2,6-NDA, aqueous dipotassium salt of 2,6-NDA, and potassium bicarbonate;
p) Separating the solid 2,6-NDA and recycling the liquid containing aqueous dipotassium salt of 2,6-NDA and potassium bicarbonate to step (n);
q) Contacting solid 2,6-NDA with water in a pipe reactor to remove traces of potassium ion impurity;
r) Separating solid 2,6-NDA and recycling water to step q).

Copending EP-A-1 212 277 offers a very efficient integrated process for producing the preferred 2,6 NDA from inexpensive olefin plant and refinery feedstock and demonstrates a number of novel aspects and advantages over any process available in the art. The process allows oxidation of crude methylnaphthalene feed, and includes improved oxidation product purification steps, including a novel hydrodebromination step, and eliminates the need to isolate a pure acid intermediate. The invention incorporates a disproportionation step, followed by new steps in separation and purification of the disproportionation product, and also demonstrates very efficient potassium recycle. Optionally, the process allows for efficient recycle of the reaction co-product and conversion into additional methylnaphthalene feed.

Methylnapthalene is fed into an oxidation reactor. For most commercial feedstock sources, the methylnaphthalene will be a mixture of two isomers, 1-methylnaphthalene and 2-methylnaphthalene. The methylnaphthalene is oxidized to a mixture of the corresponding isomers of naphthoic acid in the presence of a catalyst comprising Co, Mn and Br. The resulting crude product naphthoic acid remains in the liquid phase. The mixture of isomers of napthoic acid are recovered by an evaporative distillation which removes acetic acid, and subsequently washed with water to remove inorganic Br, phthalic acid, trimellitic acid and Co/Mn. The crude product naphthoic acid is hydrodebrominated by passing the crude product NA over a catalyst comprising palladium on carbon, the debromination taking place in the absence of solvents. The debrominated crude product NA is then washed again with water to remove residual inorganic bromine.

The crude, debrominated naphthoic acid is reacted with basic potassium in 0-50% molar excess at a temperature of about 100°C to form a concentrated solution of the dipotassium salt of the acid and to drive off carbon dioxide. The dipotassium salt of naphthoic acid and said by-products are separated. The naphthalene by-products are recycled back to the oxidation reactor. Water is added to the dipotassium salt of naphthoic acid and the aqueous solution is pumped into a reactor where water is evaporated.

The aqueous solution is then contacted with hot napthalene and the aqueous solution of salts and naphthalene is introduced into a reactor as a slurry to which has been added a catalyst comprising ZnO in an amount of 5-20% by weight.

The naphthalene slurry containing particulate salts and ZnO catalyst is reacted in a disproportionation reactor to produce 2,6 -K2NDA. The disproportionation step is optionally, and preferably, repeated in a second disproportionation reactor to improve yields.

After disproportionation the napthalene is flashed from the reaction product. The flashed naphthalene is heated and recycled for use in the evaporation of water from potassium salt, and for use as a diluent for potassium salt as it enters the disproportionation reactor. The solid product consisting of K2NDA isomers is washed and the liquid is filtered to remove catalyst and coke particles.

The liquid carrying mixed organic salts is introduced into a two-stage evaporative crystallization section where the K2NDA is selectively precipitated, the KHCO₃ is recycled, and the purified K2NDA is redissolved with additional H₂O. Then the purified K2NDA is passed through an activated carbon bed.

Next, the monopotassium salt of 2,6-NDA, (KHNDA) is selectively precipitated and the KHNDA solids are disproportionated into 2,6 -NDA and K2NDA. The product of disproportionation is centrifuged to yield a 2,6 NDA slurry, and a centrate containing predominantly 2,6 K2NDA and KHCO₃. Residual potassium is removed by passing the 2,6 NDA through a pipe reactor and washing the 2,6-NDA in water at 150°C. The process results in solid 2,6-NDA product in a water slurry.

In the process of the present invention, the solid 2 6-NDA produces by the process of copending EP-A-1 212 277 or any others which may be discovered in the future which provide polymer grade monomer is not separated as a solid, but is retained in a water slurry following purification. For example, when coupled with the process disclosed in copending EP-A-1 212 277 and EP-A-1 210 317 the final centrifuge and subsequent drying steps required to produce dry solid 2,6-NDA are eliminated by the present invention.

## Claims

1. A process for closely linking a process for producing 2,6-naphthalene dicarboxylic acid(2,6-NDA) with a process for producing polyethylene naphthalate which eliminates the need for drying 2,6-NDA, handling solid 2,6-NDA , and optimizing particle size of 2,6-NDA which comprises:
a) Pumping an aqueous slurry of polymer grade 2,6-NDA directly into a process for making PEN, either by directly pumping a stream from a 2,6 NDA process into a PEN process, or by adding water to polymer grade 2-6 - NDA prior to pumping the resulting slurry to the PEN process.
b) Removing the slurry water during the first esterification reaction at the same time the water of reaction is removed.

2. The process of Claim 1 further comprising the esterification reaction taking place in an esterification reactor designed to accommodate additional water.

3. The process of Claim 1 further comprising removing a portion of the water in the slurrry in pre-evaporator prior to the esterification reaction.

4. The process of Claim 1 wherein polymer grade 2,6-NDA is produced by:
a) Reacting a hydrocarbon stream containing predominantly methylnaphthalene with an oxygen-containing gas in the presence of a suitable solvent and catalyst to form a crude mixture of naphthoic acid (crude product NA), wherein said crude product NA remains dissolved in the solvent;
b) Recovering said crude product NA by evaporation of solvent and washing said crude product NA with water;
c) Debrominating said crude product NA by passing over a supported catalyst in the presence of hydrogen, and water-washing said crude debrominated product NA;
d) Contacting said crude, debrominated product NA with an aqueous base of potassium to extract pure NA as the aqueous potassium salt of NA;
e) Separating said aqueous potassium salt of NA from the remaining organic liquid (containing methylnaphthalene and partially oxidized reaction intermediates), and recycling said organic liquid to step a);
f) Contacting said aqueous potassium salt of NA with naphthalene vapor, adding a solid catalyst, and removing water by evaporation to form a slurry of solid potassium salt of NA and catalyst suspended in liquid naphthalene;
g) Reacting said slurry in the presence of carbon dioxide to convert solid potassium salt of NA to liquid naphthalene and solid dipotassium salt of 2,6-NDA(2,6-K2NDA);
h) Reducing the pressure to vaporize the naphthalene, and separating the solids from the naphthalene vapor by a novel separation using cyclones, recycling a portion of the naphthalene to step (f), and recovering the remainder as a product, or methylating the naphthalene via direct alkylation or transalkylation to provide additional methylnaphthalene feed for step (a);
i) Contacting the solids with water to create a mixture of aqueous potassium salts (comprising the potassium salt of NA, KNA, and the dipotassium salt of 2,6-NDA, 2,6 -K2NDA, and its isomers) and solid catalyst;
j) Separating the solid catalyst from the mixture of aqueous potassium salts and recycling it to step (f);
k) Adding aqueous potassium bicarbonate to the mixture of aqueous potassium salts and evaporating a portion of the water to selectively crystallize the dipotassium salt of 2,6-NDA as a solid, separating said solid, and recycling the remaining liquid to step (d);
l) Dissolving the solid dipotassium salt of 2,6-NDA in water;
m) Optionally passing said aqueous dipotassium salt of 2,6-NDA through an activated carbon bed to remove impurities;
n) Contacting said aqueous dipotassium salt of 2,6-NDA with carbon dioxide to create a mixture of solid monopotassium salt of 2,6-NDA and aqueous potassium bicarbonate, separating said solids, and recycling the aqueous potassium bicarbonate to step (k);
o) Contacting solid monopotassium salt of 2,6-NDA with water, optionally in the presence of carbon dioxide, to form solid 2,6-NDA, aqueous dipotassium salt of 2,6-NDA, and potassium bicarbonate;
p) Separating the solid 2,6-NDA and recycling the liquid containing aqueous dipotassium salt of 2,6-NDA and potassium bicarbonate to step (n);
q) Contacting solid 2,6-NDA with water in a pipe reactor to remove traces of potassium ion impurity;
r) Separating solid 2,6-NDA and recycling water to step o)
s) Washing the solid 2,6 NDA with additional water
t) Separating the water from the solid, producing wet polymer grade 2,6 NDA, and recycling most of the water to step q)
u) Drying the solid 2-6 NDA

5. The process of Claim 1 wherein the polymer grade 2,6-NDA is made by any process that produces polymer grade 2,6-NDA.

6. In a process for producing polyethylene naphthalate from 2,6-naphthalene dicarboxylic acid, an improvement which avoids the need for handling dry solid 2,6-naphthalene dicarboxylic acid and derivatives thereof which comprises:
a) Reacting a hydrocarbon stream containing predominantly methylnaphthalene with an oxygen-containing gas in the presence of a suitable solvent and catalyst to form a crude mixture of naphthoic acid (crude product NA), wherein said crude product NA remains dissolved in the solvent;
b) Recovering said crude product NA by evaporation of solvent and washing said crude product NA with water;
c) Debrominating said crude product NA by passing over a supported catalyst in the presence of hydrogen, and water-washing said crude debrominated product NA;
d) Contacting said crude, debrominated product NA with an aqueous base of potassium to extract pure NA as the aqueous potassium salt of NA;
e) Separating said aqueous potassium salt of NA from the remaining organic liquid (containing methylnaphthalene and partially oxidized reaction intermediates), and recycling said organic liquid to step a);
f) Contacting said aqueous potassium salt of NA with naphthalene vapor, adding a solid catalyst, and removing water by evaporation to form a slurry of solid potassium salt of NA and catalyst suspended in liquid naphthalene;
g) Reacting said slurry in the presence of carbon dioxide to convert solid potassium salt of NA to liquid naphthalene and solid dipotassium salt of 2,6-NDA(2,6-K2NDA);
h) Reducing the pressure to vaporize the naphthalene, and separating the solids from the naphthalene vapor by a novel separation using cyclones, recycling a portion of the naphthalene to step (f), and recovering the remainder as a product, or methylating the naphthalene via direct alkylation or transalkylation to provide additional methylnaphthalene feed for step (a);
i) Contacting the solids with water to create a mixture of aqueous potassium salts (comprising the potassium salt of NA, KNA, and the dipotassium salt of 2,6-NDA, 2,6 -K2NDA, and its isomers) and solid catalyst;
j) Separating the solid catalyst from the mixture of aqueous potassium salts and recycling it to step (f);
k) Adding aqueous potassium bicarbonate to the mixture of aqueous potassium salts and evaporating a portion of the water to selectively crystallize the dipotassium salt of 2,6-NDA as a solid, separating said solid, and recycling the remaining liquid to step (d);
l) Dissolving the solid dipotassium salt of 2,6-NDA in water;
m) Optionally passing said aqueous dipotassium salt of 2,6-NDA through an activated carbon bed to remove impurities;
n) Contacting said aqueous dipotassium salt of 2,6-NDA with carbon dioxide to create a mixture of solid monopotassium salt of 2,6-NDA and aqueous potassium bicarbonate, separating said solids, and recycling the aqueous potassium bicarbonate to step (k);
o) Contacting solid monopotassium salt of 2,6-NDA with water, optionally in the presence of carbon dioxide, to form solid 2,6-NDA, aqueous dipotassium salt of 2,6-NDA, and potassium bicarbonate;
p) Separating the solid 2,6-NDA and recycling the liquid containing aqueous dipotassium salt of 2,6-NDA and potassium bicarbonate to step (n);
q) Contacting solid 2,6-NDA with water in a pipe reactor to remove traces of potassium ion impurity;
r) Separating solid 2,6-NDA and recycling water to step o)
s) Washing the solid 2,6 NDA with additional water
t) Performing a crude separation of the water from the solid, producing wet polymer grade 2,6 NDA, and recycling most of the water to step q)
u) Adding water to the wet polymer grade 2,6-NDA to form a pumpable slurry,
v) Pumping the aqueous slurry directly into a process for making PEN,
w) Removing the slurry water during the first esterification reaction at the same time the water of reaction is removed.

7. The Process of Claim 6 wherein steps t) and u) are eliminated.

## Patentansprüche

1. Verfahren zum engen Koppeln eines Verfahrens zur Herstellung von 2,6-Naphthalindicarbonsäure (2,6-NDA) mit einem Verfahren zur Herstellung von Polyethylennaphthalat, welches die Notwendigkeit eines Trocknens von 2,6-NDA, Hantierens mit fester 2,6-NDA und Optimierens der Partikelgröße von 2,6-NDA beseitigt und Folgendes umfasst:
a) das direkte Einpumpen eines wässrigen Schlamms von Polymerklasse-2,6-NDA in ein Verfahren zur Herstellung von PEN, und zwar entweder durch das direkte Pumpen eines Stroms aus einem 2,6-NDA-Verfahren in ein PEN-Verfahren oder durch das Hinzufügen von Wasser zur Polymerklasse-2-6-NDA vor dem Pumpen des resultierenden Schlamms zum PEN-Verfahren.
b) das Entfernen des Schlammwassers während der ersten Veresterungsreaktion gleichzeitig mit dem Entfernen des Reaktionswassers.

2. Verfahren nach Anspruch 1, weiters umfassend die Veresterungsreaktion, welche in einem zur Aufnahme von zusätzlichem Wasser ausgelegten Veresterungsreaktor stattfindet.

3. Verfahren nach Anspruch 1, weiters umfassend das Entfernen eines Teils des Wassers im Schlamm in einem Vorverdampfer, vor der Veresterungsreaktion.

4. Verfahren nach Anspruch 1, wobei Polymerklasse-2,6-NDA hergestellt wird durch:
a) das Umsetzen eines Kohlenwasserstoffstroms, welcher überwiegend Methylnaphthalin enthält, mit einem sauerstoffhaltigen Gas in Gegenwart eines geeigneten Lösungsmittels und Katalysators, um eine Rohmischung aus Naphthoesäure (Rohprodukt NA) zu bilden, wobei das Rohprodukt NA im Lösungsmittel gelöst bleibt;
b) das Gewinnen des Rohprodukts NA durch Abdampfen des Lösungsmittels und Waschen des Rohprodukts NA mit Wasser;
c) das Entbromen des Rohprodukts NA, indem es in Gegenwart von Wasserstoff über einen Katalysator auf einem Träger geleitet wird, und das Waschen des entbromten Rohprodukts NA mit Wasser;
d) das Kontaktieren des entbromten Rohprodukts NA mit einer wässrigen Kaliumbase, um reine NA als das wässrige Kaliumsalz von NA zu extrahieren;
e) das Abtrennen des wässrigen NA-Kaliumsalzes von der restlichen organischen Flüssigkeit (enthaltend Methylnaphthalin und teilweise oxidierte Reaktionszwischenprodukte) und das Zurückführen der organischen Flüssigkeit zu Schritt a);
f) das Kontaktieren des wässrigen NA-Kaliumsalzes mit Naphthalindampf, das Hinzufügen eines festen Katalysators und das Entfernen des Wassers durch Abdampfen, um einen Schlamm aus festem NA-Kaliumsalz und einem in flüssigem Naphthalin suspendierten Katalysator zu bilden;
g) das Umsetzen des Schlamms in Gegenwart von Kohlendioxid, um festes NA-Kaliumsalz in flüssiges Naphthalin und festes Dikaliumsalz von 2,6-NDA (2,6-K2NDA) umzuwandeln;
h) das Verringern des Drucks, um das Naphthalin zu verdampfen, und das Abtrennen der Feststoffe vom Naphthalindampf mittels einer neuartigen Trennung unter Verwendung von Zyklonen, das Zurückführen eines Teils des Naphthalins zu Schritt (f) und das Gewinnen des Rests als Produkt, oder das Methylieren des Naphthalins über eine direkte Alkylierung oder Transalkylierung, um für Schritt (a) eine zusätzliche Methylnaphthalinzufuhr zu schaffen;
i) das Kontaktieren der Feststoffe mit Wasser, um eine Mischung aus wässrigen Kaliumsalzen (umfassend das Kaliumsalz von NA, KNA, sowie das Dikaliumsalz von 2,6-NDA, 2,6-K2NDA, und dessen Isomere) und einem festen Katalysator zu schaffen;
j) das Abtrennen des festen Katalysators von der Mischung aus wässrigen Kaliumsalzen und das Zurückführen desselben zu Schritt (f);
k) das Hinzufügen von wässrigem Kaliumbicarbonat zur Mischung aus wässrigen Kaliumsalzen und das Abdampfen eines Teils des Wassers, um das Dikaliumsalz von 2,6-NDA selektiv als Feststoff zu kristallisieren, das Abtrennen des Feststoffs und das Zurückführen der restlichen Flüssigkeit zu Schritt (d);
l) das Auflösen des festen Dikaliumsalzes von 2,6-NDA in Wasser;
m) gegebenenfalls das Leiten des wässrigen Dikaliumsalzes von 2,6-NDA durch ein Aktivkohlebett, um Verunreinigungen zu entfernen;
n) das Kontaktieren des wässrigen Dikaliumsalzes von 2,6-NDA mit Kohlendioxid, um eine Mischung aus festem Monokaliumsalz von 2,6-NDA und wässrigem Kaliumbicarbonat zu schaffen, das Abtrennen der Feststoffe und das Zurückführen des wässrigen Kaliumbicarbonats zu Schritt (k);
o) das Kontaktieren des festen Monokaliumsalzes von 2,6-NDA mit Wasser, gegebenenfalls in Gegenwart von Kohlendioxid, um feste 2,6-NDA, wässriges Dikaliumsalz von 2,6-NDA und Kaliumbicarbonat zu bilden;
p) das Abtrennen der festen 2,6-NDA und das Zurückführen der Flüssigkeit, die wässriges Dikaliumsalz von 2,6-NDA und Kaliumbicarbonat enthält, zu Schritt (n);
q) das Kontaktieren von fester 2,6-NDA mit Wasser in einem Rohrreaktor, um die Spuren einer Verunreinigung mit Kaliumionen zu entfernen;
r) das Abtrennen von fester 2,6-NDA und das Zurückführen von Wasser zu Schritt o);
s) das Waschen der festen 2,6-NDA mit zusätzlichem Wasser;
t) das Abtrennen des Wassers vom Feststoff, das Herstellen von nasser Polymerklasse-2,6-NDA und das Zurückführen eines Großteils des Wassers zu Schritt q);
u) das Trocknen der festen 2-6-NDA.

5. Verfahren nach Anspruch 1, wobei die Polymerklasse-2,6-NDA durch irgendein Verfahren, welches Polymerklasse-2,6-NDA produziert, hergestellt wird.

6. Verfahren zur Herstellung von Polyethylennaphthalat aus 2,6-Naphthalindicarbonsäure, eine Verbesserung, welche die Notwendigkeit des Hantierens mit trockener fester 2,6-Naphthalindicarbonsäure und deren Derivaten vermeidet und Folgendes umfasst:
a) das Umsetzen eines Kohlenwasserstoffstroms, welcher überwiegend Methylnaphthalin enthält, mit einem sauerstoffhaltigen Gas in Gegenwart eines geeigneten Lösungsmittels und Katalysators, um eine Rohmischung aus Naphthoesäure (Rohprodukt NA) zu bilden, wobei das Rohprodukt NA im Lösungsmittel gelöst bleibt;
b) das Gewinnen des Rohprodukts NA durch Abdampfen des Lösungsmittels und Waschen des Rohprodukts NA mit Wasser;
c) das Entbromen des Rohprodukts NA, indem es in Gegenwart von Wasserstoff über einen Katalysator auf einem Träger geleitet wird, und das Waschen des entbromten Rohprodukts NA mit Wasser;
d) das Kontaktieren des entbromten Rohprodukts NA mit einer wässrigen Kaliumbase, um reine NA als das wässrige Kaliumsalz von NA zu extrahieren;
e) das Abtrennen des wässrigen NA-Kaliumsalzes von der restlichen organischen Flüssigkeit (enthaltend Methylnaphthalin und teilweise oxidierte Reaktionszwischenprodukte) und das Zurückführen der organischen Flüssigkeit zu Schritt a);
f) das Kontaktieren des wässrigen NA-Kaliumsalzes mit Naphthalindampf, das Hinzufügen eines festen Katalysators und das Entfernen des Wassers durch Abdampfen, um einen Schlamm aus festem NA-Kaliumsalz und einem in flüssigem Naphthalin suspendierten Katalysator zu bilden;
g) das Umsetzen des Schlamms in Gegenwart von Kohlendioxid, um festes NA-Kaliumsalz in flüssiges Naphthalin und festes Dikaliumsalz von 2,6-NDA (2,6-K2NDA) umzuwandeln;
h) das Verringern des Drucks, um das Naphthalin zu verdampfen, und das Abtrennen der Feststoffe vom Naphthalindampf mittels einer neuartigen Trennung unter Verwendung von Zyklonen, das Zurückführen eines Teils des Naphthalins zu Schritt (f) und das Gewinnen des Rests als Produkt, oder das Methylieren des Naphthalins über eine direkte Alkylierung oder Transalkylierung, um für Schritt (a) eine zusätzliche Methylnaphthalinzufuhr zu schaffen;
i) das Kontaktieren der Feststoffe mit Wasser, um eine Mischung aus wässrigen Kaliumsalzen (umfassend das Kaliumsalz von NA, KNA, sowie das Dikaliumsalz von 2,6-NDA, 2,6-K2NDA, und dessen Isomere) und einem festen Katalysator zu schaffen;
j) das Abtrennen des festen Katalysators von der Mischung aus wässrigen Kaliumsalzen und das Zurückführen desselben zu Schritt (f);
k) das Hinzufügen von wässrigem Kaliumbicarbonat zur Mischung aus wässrigen Kaliumsalzen und das Abdampfen eines Teils des Wassers, um das Dikaliumsalz von 2,6-NDA selektiv als Feststoff zu kristallisieren, das Abtrennen des Feststoffs und das Zurückführen der restlichen Flüssigkeit zu Schritt (d);
l) das Auflösen des festen Dikaliumsalzes von 2,6-NDA in Wasser;
m) gegebenenfalls das Leiten des wässrigen Dikaliumsalzes von 2,6-NDA durch ein Aktivkohlebett, um Verunreinigungen zu entfernen;
n) das Kontaktieren des wässrigen Dikaliumsalzes von 2,6-NDA mit Kohlendioxid, um eine Mischung aus festem Monokaliumsalz von 2,6-NDA und wässrigem Kaliumbicarbonat zu schaffen, das Abtrennen der Feststoffe und das Zurückführen des wässrigen Kaliumbicarbonats zu Schritt (k);
o) das Kontaktieren des festen Monokaliumsalzes von 2,6-NDA mit Wasser, gegebenenfalls in Gegenwart von Kohlendioxid, um feste 2,6-NDA, wässriges Dikaliumsalz von 2,6-NDA und Kaliumbicarbonat zu bilden;
p) das Abtrennen der festen 2,6-NDA und das Zurückführen der Flüssigkeit, die wässriges Dikaliumsalz von 2,6-NDA und Kaliumbicarbonat enthält, zu Schritt (n);
q) das Kontaktieren von fester 2,6-NDA mit Wasser in einem Rohrreaktor, um die Spuren einer Verunreinigung mit Kaliumionen zu entfernen;
r) das Abtrennen von fester 2,6-NDA und das Zurückführen von Wasser zu Schritt o);
s) das Waschen der festen 2,6-NDA mit zusätzlichem Wasser;
t) das Durchführen einer groben Abtrennung des Wassers vom Feststoff, das Herstellen von nasser Polymerklasse-2,6-NDA und das Zurückführen eines Großteils des Wassers zu Schritt q);
u) das Hinzufügen von Wasser zur nassen Polymerklasse-2,6-NDA, um einen pumpfähigen Schlamm zu bilden;
v) das direkte Einpumpen des wässrigen Schlamms in ein Verfahren zur Herstellung - von PEN;
w) das Entfernen des Schlammwassers während der ersten Veresterungsreaktion gleichzeitig mit dem Entfernen des Reaktionswassers.

7. Verfahren nach Anspruch 6, wobei die Schritte t) und u) weggelassen werden.

## Revendications

1. Procédé pour l'association étroite d'un procédé pour obtenir de l'acide 2,6-naphtalène dicarboxylique (2,6-NDA) avec un procédé pour obtenir du polyéthylène naphtalate qui élimine le besoin de sécher le 2,6-NDA, de manipuler le 2,6-NDA solide, et d'optimiser la taille de particule du 2,6-NDA qui comprend les étapes consistant à :
a) alimenter directement en un mélange aqueux de 2,6-NDA de qualité polymère un procédé pour fabriquer du PEN, soit en pompant directement depuis un courant provenant d'un procédé de 2,6-NDA dans un procédé de PEN, soit en ajoutant de l'eau à du 2,6-NDA de qualité polymère avant de pomper le mélange résultant dans le procédé PEN.
b) éliminer l'eau du mélange durant la première réaction d'estérification en même temps que l'eau de réaction est éliminée.

2. Procédé selon la revendication 1, selon lequel en outre la réaction d'estérification a lieu dans un réacteur d'estérification conçu pour recevoir de l'eau additionnelle.

3. Procédé selon la revendication 1, selon lequel en outre on élimine une partie de l'eau du mélange dans un préconcentrateur avant la réaction d'estérification.

4. Procédé selon la revendication 1 dans lequel le 2,6-NDA de qualité polymère est obtenu par :
a) réaction d'un courant d'hydrocarbure contenant majoritairement du méthylnaphtalène avec un gaz contenant de l'oxygène en présence d'un solvant adapté et d'un catalyseur pour former un mélange brut d'acide naphtoïque (produit NA brut), dans lequel ledit produit NA brut reste dissous dans le solvant ;
b) récupération dudit produit NA brut par évaporation du solvant et lavage dudit produit NA brut avec de l'eau ;
c) débromuration dudit produit NA brut par passage sur un catalyseur supporté en présence d'hydrogène et lavage à l'eau dudit produit NA débromé brut;
d) mise en contact dudit produit NA débromé, brut avec une base aqueuse de potassium pour extraire le NA pur sous forme du sel de potassium aqueux de NA ;
e) séparation dudit sel de potassium aqueux de NA du liquide organique restant (contenant du méthylnaphtalène et des intermédiaires de réaction partiellement oxydés), et recyclage dudit liquide organique vers l'étape a)
f) mise en contact dudit sel de potassium aqueux de NA avec de la vapeur de naphtalène, ajout d'un catalyseur solide, et élimination de l'eau par évaporation pour former un mélange de sel de potassium solide de NA et de catalyseur mis en suspension dans le naphtalène liquide ;
g) réaction de ladite suspension en présence de dioxyde de carbone pour convertir le sel de potassium solide de NA en naphtalène liquide et en sel de dipotassium solide de 2,6-NDA (2,6-K₂NDA) ;
h) réduction de la pression pour vaporiser le naphtalène, et séparation des solides de la vapeur de naphtalène par une nouvelle séparation utilisant des cyclones, recyclage d'une partie du naphtalène vers l'étape (f), et récupération du reste comme produit, ou méthylation du naphtalène par alkylation ou transalkylation directe pour fournir une alimentation additionnelle de méthylnaphtalène pour l'étape (a) ;
i) mise en contact des solides avec de l'eau pour créer un mélange de sels de potassium aqueux (comprenant le sel de potassium du NA, le KNA, et le sel de dipotassium du 2,6-NDA, le 2,6-K₂NDA, et ses isomères) et de catalyseur solide ;
j) séparation du catalyseur solide du mélange de sels de potassium aqueux et son recyclage vers l'étape (f) ;
k) ajout de bicarbonate de potassium aqueux au mélange de sels de potassium aqueux et évaporation d'une partie de l'eau pour cristalliser de façon sélective le sel de dipotassium du 2,6-NDA sous forme d'un solide, séparation dudit solide, et recyclage du liquide restant vers l'étape (d) ;
l) dissolution du sel de dipotassium solide du 2,6-NDA dans de l'eau ;
m) éventuellement passage de ladite solution de sel de dipotassium aqueux de 2,6-NDA au travers d'un lit de charbon activé pour éliminer les impuretés ;
n) mise en contact dudit sel de dipotassium aqueux de 2,6-NDA avec du dioxyde de carbone pour créer un mélange de sel solide de monopotassium de 2,6-NDA et de bicarbonate de potassium aqueux, séparation des solides, et recyclage du bicarbonate de potassium aqueux vers l'étape (k) ;
o) mise en contact du sel de monopotassium solide de 2,6-NDA avec de l'eau, éventuellement en présence de dioxyde de carbone, pour former du 2,6-NDA solide, du sel de dipotassium aqueux de 2,6-NDA, et du bicarbonate de potassium ;
p) séparation du 2,6-NDA solide et recyclage du liquide contenant le sel de dipotassium aqueux de 2,6-NDA et le bicarbonate de potassium vers l'étape (n) ;
q) mise en contact du 2,6-NDA solide avec de l'eau dans un réacteur tubulaire pour éliminer les traces d'impureté d'ion potassium ;
r) séparation du 2,6-NDA solide et recyclage de l'eau vers l'étape o) ;
s) lavage du 2,6-NDA solide avec de l'eau additionnelle ;
t) séparation de l'eau du solide, obtention de 2,6-NDA de qualité polymère humide et recyclage de la majeure partie de l'eau vers l'étape q) ;
u) séchage du 2,6-NDA solide ;

5. Procédé selon la revendication 1, dans lequel le 2,6-NDA de qualité polymère est fabriqué par n'importe quel procédé qui produit du 2,6-NDA de qualité polymère.

6. Dans un procédé pour produire du polyéthylène naphtalate à partir d'acide 2,6-naphtalène dicarboxylique, un perfectionnement qui évite le besoin de manipuler de l'acide 2,6-naphtalène dicarboxylique solide sec et des dérivés de celui-ci ledit perfectionnement comprenant les étapes consistant à :
a) faire réagir un courant d'hydrocarbure contenant majoritairement du méthylnaphtalène avec un gaz contenant de l'oxygène en présence d'un solvant adapté et d'un catalyseur pour former un mélange brut d'acide naphtoïque (produit NA brut), dans lequel ledit produit NA brut reste dissous dans le solvant ;
b) récupérer ledit produit NA brut par évaporation du solvant et laver ledit produit NA brut avec de l'eau ;
c) débromurer ledit produit NA brut par passage sur un catalyseur supporté en présence d'hydrogène, et lavage à l'eau dudit produit NA débromé brut ;
d) mettre en contact ledit produit NA débromé, brut avec une base aqueuse de potassium pour extraire le NA pur sous forme du sel de potassium aqueux de NA ;
e) séparer ledit sel de potassium aqueux de NA du liquide organique restant (contenant du méthylnaphtalène et des intermédiaires de réaction partiellement oxydés), et recycler ledit liquide organique vers l'étape a) ;
f) mettre en contact ledit sel de potassium aqueux de NA avec de la vapeur de naphtalène, ajouter un catalyseur solide, et éliminer l'eau par évaporation pour former un mélange de sel de potassium solide de NA et de catalyseur mis en suspension dans le naphtalène liquide ;
g) faire réagir ladite suspension en présence de dioxyde de carbone pour convertir le sel de potassium solide de NA en naphtalène liquide et en sel de dipotassium solide de 2,6-NDA (2,6-K₂NDA) ;
h) réduire la pression pour vaporiser le naphtalène, et séparer les solides de la vapeur de naphtalène par une nouvelle séparation utilisant des cyclones, recycler une partie du naphtalène vers l'étape (f), et récupérer le reste comme produit, ou méthyler le naphtalène par alkylation ou transalkylation directe pour fournir une alimentation additionnelle de méthylnaphtalène pour l'étape (a) ;
i) mettre en contact les solides avec de l'eau pour créer un mélange de sels de potassium aqueux (comprenant le sel de potassium du NA, le KNA, et le sel de dipotassium du 2,6-NDA, le 2,6-K₂NDA, et ses isomères) et de catalyseur solide ;
j) séparer le catalyseur solide du mélange de sels de potassium aqueux et le recycler vers l'étape (f) ;
k) ajouter du bicarbonate de potassium aqueux au mélange de sels de potassium aqueux et évaporer une partie de l'eau pour cristalliser de façon sélective le sel de dipotassium du 2,6-NDA sous forme d'un solide, séparer ledit solide, et recycler le liquide restant vers l'étape (d) ;
l) dissoudre le sel de dipotassium solide du 2,6-NDA dans de l'eau ;
m) éventuellement faire passer ladite solution de sel de dipotassium aqueux de 2,6-NDA au travers d'un lit de charbon activé pour éliminer les impuretés ;
n) mettre en contact ledit sel de dipotassium aqueux de 2,6-DNA avec du dioxyde de carbone pour créer un mélange de sel solide de monopotassium de 2,6-DNA et de bicarbonate de potassium aqueux, séparer les solides, et recycler le bicarbonate de potassium aqueux vers l'étape (k) ;
o) mettre en contact le sel de monopotassium solide de 2,6-NDA avec de l'eau, éventuellement en présence de dioxyde de carbone, pour former du 2,6-NDA solide, du sel de dipotassium aqueux de 2,6-NDA, et du bicarbonate de potassium ;
p) séparer le 2,6-NDA solide et recycler le liquide contenant le sel de dipotassium aqueux de 2,6-NDA et le bicarbonate de potassium vers l'étape (n) ;
q) mettre en contact le 2,6-NDA solide avec de l'eau dans un réacteur tubulaire pour éliminer les traces d'impureté d'ion potassium ;
r) séparer le 2,6-NDA solide et recycler l'eau vers l'étape o) ;
s) laver le 2,6-NDA solide avec de l'eau additionnelle;
t) réaliser une séparation sommaire de l'eau du solide, pour obtenir du 2,6-NDA de qualité polymère humide, et recycler la majeure partie de l'eau vers l'étape q) ;
u) ajouter de l'eau au 2,6-NDA de qualité polymère humide pour former un mélange pouvant être pompé ;
v) alimenter directement en mélange aqueux, par pompage dans un procédé pour fabriquer le PEN ;
w) éliminer l'eau du mélange durant la première réaction d'estérification en même temps que l'eau de réaction est éliminée.

7. Procédé selon la revendication 6, dans lequel les étapes t) et u) sont éliminées.
